# EUROPEAN PATENT SPECIFICATION

(11) **EP 1 351 666 B1**
(45) Date of publication and mention of the grant of the patent: **27.02.2008**
(21) Application number: 02710900.8
(22) Date of filing: 18.01.2002
(51) Int. Cl.: A61K 9/14, A61K 9/12, A61J 3/02

(54) **A METHOD FOR THE PREPARATION OF NANOPARTICLES**
VERFAHREN ZUR HERSTELLUNG VON NANOPARTIKELN
PROCEDE DE PREPARATION DE NANOPARTICULES

(30) Priority: 18.01.2001 FI 20010115
(43) Date of publication of application: 15.10.2003
(73) Proprietor: ORION CORPORATION, 02200 Espoo (FI)
(72) Inventor: WATANABE, Wiwik, San Jose, CA 95112 (US); KAUPPINEN, Esko, FIN-00730 Helsinki (FI); AHONEN, Petri, FIN-09630 Koisjärvi (FI); BROWN, David, FIN-00500 Helsinki (FI); MUTTONEN, Esa, FIN-02210 Espoo (FI)
(74) Representative: Sexton, Jane Helen
(86) International application number: PCT/FI2002/000042
(87) International publication number: WO 2002/056866

(56) References cited:
- EP-A1- 0 504 760
- EP-A1- 0 600 528
- EP-A2- 0 499 299
- US-A- 6 051 257

## Description

### Field of the invention

The present invention relates to free nano-sized particles of active agents and to the method for preparing such particles. The method is particularly useful in the preparation of nano-sized particles of therapeutically active agents such particles being suitable for e.g. oral, pulmonary or transdermal delivery.

### Background of the invention

Increased drug dissolution rate is often desirable, particularly when bioavailability (the amount of drug available in the targeted site(s) after administration) problems are encountered. One way to increase the dissolution rate of an active material is by increasing the specific surface area of a given particle. This can be achieved by reducing the particle size. Furthermore, reducing the particle size to nanometers increases the accessability of the drugs to the targeted sites.

Efforts have been made to generate drug particles with size down to tens of nanometers. Dry and wet milling techniques have been widely used to reduce the particle size to tens of nanometers (US 4,107,288, US 5,534,270, US 6,045,829). However, the nano-sized particles cause cake formations in the grinding materials and milling chambers thereby significantly reducing the size reduction efficiency of the process. This results in broad particle size distribution and non-uniformity in particle size. Furthermore, the milling process can introduce changes in particle morphology, damage in particle crystalline structures, variability of particle properties among different batches, and possibly contamination, which is undesirable in pharmaceutical material productions.

Other approaches to produce nanoparticles include a method described in WO 97/13503. The method involves combining the agent and matrix materials to form a composite mixture, which is then spray dried to form a nanocomposite. Accordingly, the method produces composite particles wherein nano-sized drug particles are distributed and embedded within the surface structure of the matrix constituents. However, addition of other materials in drugs are usually not desired, especially when the additional materials may induce side effects upon administration into the body.

US-A-6051257 describes a powder batch of pharmaceutically active particles and an aerosol-based method for making them.

The present invention aims to provide a simple and efficient method which is able to produce free nano-sized particles of an active agent with consistent and controlled particle properties, including particle size and size distribution, shape, crystallinity, polymorphic phase, surface roughness and chemical purity. In particular, the aim is to provide nano-sized particles, which are not strongly adhered to or embedded within a solid matrix material as in WO 97/13503. Such particles would be particularly well suited for e.g. oral, pulmonary, and transdermal drug delivery.

### Summary of the Invention

It has been found that it is possible to obtain free uncharged, spherical and crystalline nano-sized particles of active agents with a narrow aerodynamic particle size distribution, using an aerosol flow reactor method. The active agent is preferably a therapeutic, cosmetic or diagnostic agent. The method is particularly useful in the preparation of free nano-sized particles of therapeutically active agents. The size of the resulted particles may be in the range from a few micrometers to as small as 3 nm. The particles exhibit improved dispersibility, good stability as a result of their crystalline nature, and more accurate delivery of the active agents into the targeted sites. Moreover, the method of the invention provides a high purity product since the product purity only depends on the purity of solution precursors. The method is simple and can be easily scaled-up to higher production rates.

In one aspect, the present invention provides a method for preparing free nano-sized particles of an active agent, comprising the steps of:
providing a liquid feed stock comprising an active agent or combination of two or more active agents;
atomising the liquid feed stock to create droplets;
suspending said droplets in a carrier gas;
passing said carrier gas and droplets suspended therein through a heated tube flow reactor under predetermined residence time and temperature history; and
collecting the nano-sized particles produced.

The nano-sized particles obtained are particularly suitable for use in pharmaceutical, cosmetic or diagnostic compositions. For example, the nano-sized drug particles can be used in dry powders or in powders dispersed in liquids or colloidal suspensions for pulmonary drug delivery, tablets, capsules, mixtures, emulsions or syrups for oral administration or for patches and the like for transdermal drug delivery.

The mean mass aerodynamic diameter of the particles is between 10 and 200 nm, typically between 20 and 200 nm, more typically between 30 and 100 nm. The aerodynamic particle size distribution of the particles is generally between 3 and 5000 nm, typically between 10 and 1000 nm, more typically between 20 and 200 nm.

The term "free nano-sized particles" means here nano-sized particles, which are not strongly adhered to or embedded within a solid matrix material. A "free nano-sized particle" produced according to the method of the invention is an individual particle possibly loosely agglomerated with other nano-sized particles.

### Brief Description of the Drawings

Figures 1a and 1b are schematic diagrams showing parts of the apparatus used in the method of the invention.
Figure 2 is normalized number size distribution of beclomethasone dipropionate particles produced.
Figures 3a and 3b show TEM images of beclomethasone dipropionate particles.
Figure 4 shows a diffractogram of beclomethasone dipropionate particle.

### Detailed Description of the Invention

The present invention employs an aerosol flow reactor method (aerosol synthesis method). It is a one-step continuous process, which can directly produce particles within a desirable size range. The method of the present invention differs significantly from the conventional spray drying process. In spray drying, hot gas is used as a source of energy to evaporate the solvent The spray drying chamber is only used as a place for the heat transfer between gas streams to occur. The chamber itself is not heated. The temperature of the gas changes across the chamber as heat transfer occurs between the cold feed and the hot gas. Furthermore, the evaporation is typically so rapid that it is not easy to properly control the temperature history and the residence time of each droplet and product particle. Thus, the crystallization can not be easily controlled, and the particles formed are typically amorphous.

In the method of the invention, the droplets containing the active agent are suspended in the carrier gas before they are fed into the tubular flow reactor, which is maintained at a constant temperature. The carrier gas flows evenly m the tubular reactor with a constant rate, and controlled temperature and flow field. Therefore, the temperature history and the residence time of each droplet and product particle can be accurately controlled and excellent uniformity of the particles can be ensured. Accordingly, the method provides better control of the droplet size distribution, and thus the particle size distribution. Furthermore, in contrast to spray drying, the method of the invention allows essentially complete crystallization of the particles.

The method of the invention comprises generally the following steps:
providing a liquid feed stock comprising an active agent or combination of two or more active agents;
atomising the liquid feed stock to create droplets;
suspending said droplets in a carrier gas;
passing said carrier gas and droplets suspended therein through a heated tube flow reactor under predetermined residence time and temperature history; and
collecting the nano-sized particles produced.

The liquid feed stock is prepared by mixing the active agent with a suitable liquid solvent. The liquid feed stock may be in the form of a solution, suspension, dispersion, gel, emulsion, slurry or the like, and is preferably homogenous to ensure uniform distribution of the components in the mixture. The liquid feed stock in the form of a solution is preferred. Various solvents may be employed in the preparation of the a liquid feed stock, including but not limited to, water, hydrocarbons, halogenated hydrocarbons, alchohols, ketones and the like. Examples of suitable solvents include water, hexane, perfluorohexane, ethanol, methanol, acetone, chloroform, methylene chloride and combinations thereof.

In case the liquid feed stock is a solution, the active agent should be sufficiently soluble in the solvent so as to obtain, from the atomized droplets of the liquid feed stock, uniform particles with the desired particle size and size distribution. The total solids dissolved may be present in wide range of concentrations, typically from about 0.01 % to about 25 % by weight, preferably from about 1 % to about 5 % by weight. A liquid feed stock containing a relatively low concentration of solids results in particles having relatively small diameter. The finding of suitable liquid feed stock concentrations for each active agent/solvent combinations is considered to be routine to one skilled in the art.

Any of a variety of active agents, and in particular therapeutically active agents, can be used in the method of the invention. Particularly suitable agents include, but are not limited to, anti-inflammatory agents such as aspirin, naproxen, ibuprofen, beclomethasone, indomethacin and diclofenac and their salts, bronchodilator agents such as formoterol, salbutamol, terbutaline, procaterol, and salmeterol and their salts, antibiotic agents such as penicillins, tetracillins, aminiglycoside, antiviral agents, immunosuppresive agents, immunostimulatory agents, antifungal agents, anesthetic agents, antioxidants, antidiabetic agents, vitamins, hormones, antiepilectic agents, muscle relaxants, anti-HIV agents, anticancer agents, stimulants, cough supressants, pain controls, smoking cessation agents, anti alcohol abuse agents and combinations thereof. Such therapeutic agents can be peptides, proteins, nucleic acids, carbohydrates, lipids, steroids, radioactive compounds, and the like, and the combinations thereof.

The method of the invention can be also used to produce nano-sized particles of cosmetic and diagnostic agents. If desired, particles of e.g. photochemical, catalyst, fertilizer, pigment, propellant, food, explosive or agriculture agents can be also prepared.

The liquid feed stock is atomized to create droplets in a suitable atomizer, which are well known in the art, such as a spray nozzle (e.g. a two fluid nozzle), an air assisted or air blast nebuliser, a spinning disc, a pressurised liquid atomizer, an ultrasonic nebulizer, electro spraying, vibrating orifice aerosol generator (VOAG) or rotating aerosol generator. Ultrasonic and air-assisted nebulizers are preferred. Examples of the devices used in this process include ultrasonic and air-assisted aerosol generators sold under trademarks GAPUSOL 9001 and TSI 3076, respectively. While there are no special restrictions placed on the atomisers used in the process, it is recommended to use an atomiser that can produce uniform droplets of constant composition and with a narrow droplet size distribution. Such devices are suitable to produce nanodrugs of controlled composition and particle size range suitable for drug delivery.

The size of the atomized droplets are generally smaller than about 10 µm, preferably smaller than about 5 µm, preferably below 1 µm, depending on the composition of the liquid feed stock and the desired final particle size.

The droplets of the liquid feed stock are suspended in a carrier gas before passing through a heated tubular flow reactor. The carrier gas is preferably inert with respect to the drug molecules and the solvent. It is recommended to use nitrogen gas or other inert gases. Alternatively, the gas may include a component contributing to the formation of particles. However, an inert gas is preferred.

The droplets suspended in the carrier gas are passed through a tubular flow reactor, which is maintained at a constant temperature or divided into several heating zones. Constant temperature of the reactor is preferred. The temperature and the flow rate of the carrier gas are adjusted to evaporate the solvent and to allow the crystallisation process to complete. Because the droplets are already suspended in the carrier gas when fed into the reactor (i.e. the droplet generation and flow reactor are separated), the temperature history and residence time of each droplet and product particle can be properly controlled. Therefore, excellent uniformity of the resulted particles and narrow particle size distribution can be ensured.

The particles formed are then collected using an electrostatic precipitator, a cyclone, a planar filter (e.g. nylon, teflon, etc.) or other particle collecting devices.

The aerosol flow reactor conditions are preferably selected such that crystalline or amorphous (depending on the application), essentially spherical particles of homogeneous constituents having a narrow particle size distribution and rough surfaces are formed

The mean mass aerodynamic diameter of the particles is generally between 10 and 200 nm, typically between 20 and 200 nm, more typically between 30 and 200 nm. It is particularly preferred that the mean mass aerodynamic diameter of the particles is less than 500 nm, more preferably less than 200 nm, and most preferably less than 100 nm.

The aerodynamic particle size distribution of the particles is generally between 3 and 5000 nm, typically between 5 and 1000 mn, more typically between 10 and 200 nm. It is preferred that more than about 95 % of the mass is in the particles having a diameter of 1000 nm or less, particularly 500 nm or less. It is also preferred that at least about 10 %, particularly at least about 20 %, most preferably at least about 50 %, of the mass is in the particles having a diameter af less than 100 nm.

Preferably, the particles have a narrow particle size distribution with geometric standard deviation less than 2, preferably less than 1.5, most preferably less than 1.3.

The particles of the invention have the relative degree of crystallinity preferably 50 % or higher, more preferably 90 % or higher, most preferably 99 % or higher. The relative degree of crystallinity can be estimated e.g. based on TEM electron diffraction patterns or x-ray powder diffraction patterns.

Typically, the particles obtained are essentially spherical, i.e. the spherical form is consistent and apparent when examined under a scanning electron microscope or transmission electron microscope. Typically, the particles obtained have rough surface, i.e. the roughness is consistent over the entire surface of the particle, apparent when examined under the scanning electron microscope or transmission electron microscope, and the ratio of the maximum and minimum diameter of the particle is between 1.001-1.5, preferably between 1.002 -1.2, more preferably between 1.01-1.1.

According to one preferred embodiment of the invention, the active agents constitute at least 90 w-%, preferably at least 95 w-%, more preferably at least 99 w-%, of the total weight of particles, and most preferably the particles are essentially free from other material than the active agents.

However, and according to another preferred embodiment of the invention, various additives known in the art may be additionally incorporated in the particles together with the active agents. Such additives include e.g. diluents, carriers and stabilizers and the like. In such case the additives are included in the liquid feed stock of the process together with the active agents.

The particle size may be controlled to any desired particle size ranges by selection of the atomizer and its operating conditions and concentration and composition of the liquid feed stock, or employing a droplet size modification apparatus (e.g. impactor or virtual impactor, or using size selective collection particles, e.g. cyclone) upstream or downstream of the flow reactor.

For the tubular flow reactor, while there are no particular restrictions, it is recommended to use a vertical, rather than horizontal configuration in order to minimise buoyancy effects and related losses due to recirculating flow. To ensure uniform temperature and flow fields in the hot zone of the reactor, CFD (Computational Fluid Dynamics) calculations have shown that it is preferable that the aerosol flows against gravity. While there is no particular restrictions placed on the orientation, it has been found that flow in any other direction tends to produce undesirable reactor conditions. The reactor tube is preferably maintained at a uniform reactor wall temperature during the process. The reactor temperature is set such that the materials being processed do not decompose. Typically the selected reactor temperature is within the range of about 30 to 300 °C. The reactor tube may be maintained at a constant temperature or divided into different temperature zones, over its entire length. The constant temperature is preferred.

While there are no particular restrictions placed on the particle collection, it is recommended to use a system that can be heated to prevent the re-condensation process. Electrostatic precipitators (ESP), cyclones, filters and/or wet particle collection such as wet ESP can be used for this purpose. Accordingly, the particle collection system and the line from the flow reactor outlet to the particle collection system are preferably heated to a temperature above the boiling point of the solution to prevent the re-condensation process to occur.

Various compositions comprising nano-sized particles of the invention can be prepared according to the methods known in the art. The particles obtained are generally well suited for use as tablet, capsule, powder for inhalable drugs, powder dispersed in liquid or colloidal suspension for transdermal drug delivery or pulmonary delivery using pMDIs or nebulizers. Nano-sized particles show improved dissolution rate in-vitro and bioavailability in-vivo, dispersibility and stability. The particles may optionally be combined with a pharmaceutically acceptable carrier materials or excipients, which are suitable for the drug delivery. Such carriers may be used simply as bulking agents, to improve the dispersibility of the powder, as controlled-release, or as surface stabilizers.

The invention is further illustrated by the following experiments, which are not meant to limit the scope of the invention.

### Example 1. Nano-sized beclomethasone dipropionate particles

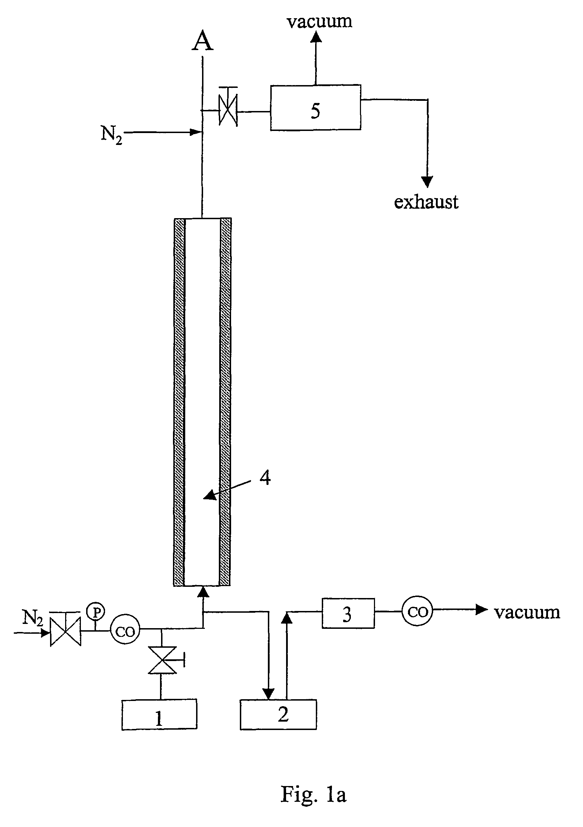

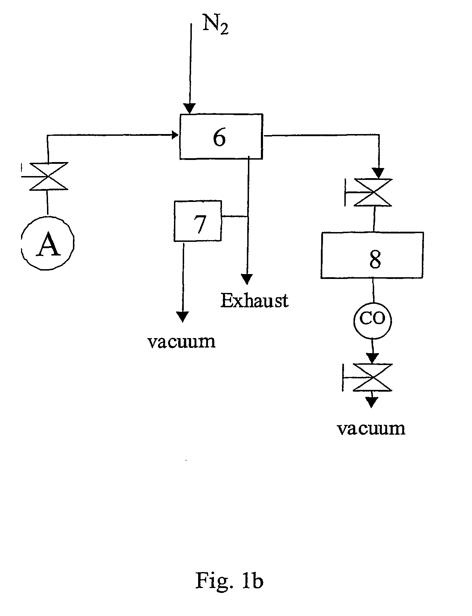

Figure 1 a shows the experimental set-up of the particle synthesis, and Fig. 1b shows optional configurations used for particle analysis. Beclomethasone dipropionate is an anti-inflammatory glucocorticosteroid, used for asthma therapy. The beclomethasone dipropionate liquid feed stock was prepared by dissolving 0.25 g of beclomethasone dipropionate powder in 1 liter of ethanol (99.5%) at room temperature. The liquid feed stock described above was atomised using an air-assisted aerosol generator (1), sold under trade mark TSI 3076, operating at solution feed rate of 0.1 to 2 ml/min. The resulting droplets, which were suspended into carrier gas, were then passed through a heated tube flow reactor (4). Nitrogen gas is preferably used as a carrier gas. The carrier gas flow rate to the reactor was Kept constant at 1.5 1/min. The excess gas was drawn to a dripping bottle (2) and then directed to a vacuum via a filter (3). A heated vertical laminar flow reactor tube (4) was used to evaporate the solvent from the droplets. The reactor tube is made of stainless steel, with an inner diameter and a heated length of 30 and 800 mm, respectively. The reactor temperature was set at 150 °C. Particle residence time in the heated zone under the selected process conditions is preferably between 2 and 30 seconds, with total residence time preferably between 12 and 50 seconds. The flow reactor design and the experimental conditions were optimised using CFD calculations, to ensure sufficient uniformity of the temperature and velocity fields and that there are no recirculating regions in the heated zone. The resulting particles were then collected using teflon filter (5) for further particle chracterization.

### Characterisation

### i. Particle size analysis

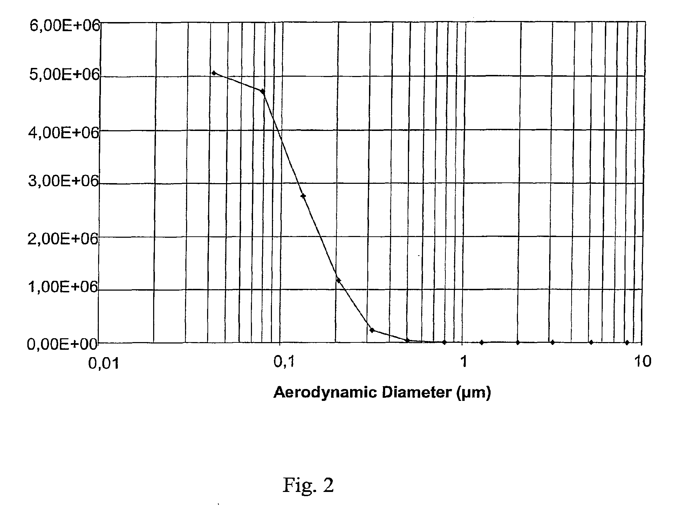

Referring now to Fig. 1b, the particle size distribution was measured by an electrical low pressure impactor (8) (ELPI) connected to a vacuum. The particles exiting the tubular reactor were passed into a diluter (6), with a dilution ratio of 1:10, before entering the ELPI. Some particles exiting from the first diluter were sampled directly into a TEM grid (7) via a combination electrostatic precipitator connected to a vacuum, for morphology analysis. To minimize temperature gradient, and thus to reduce the moisture condensation, the diluter, the line to the diluter and the gas line into the diluter were layered with heating elements that were kept also at 100°C. The gravimetric measurement shows that a narrow particle size distribution within the range of aerodynamic size of interest, i.e. at around 30-200 nm, was obtained, as shown in Figure 2 (dN/dlogDₐₑ vs. µm).

### ii. Particle morphology and crystallinity analysis

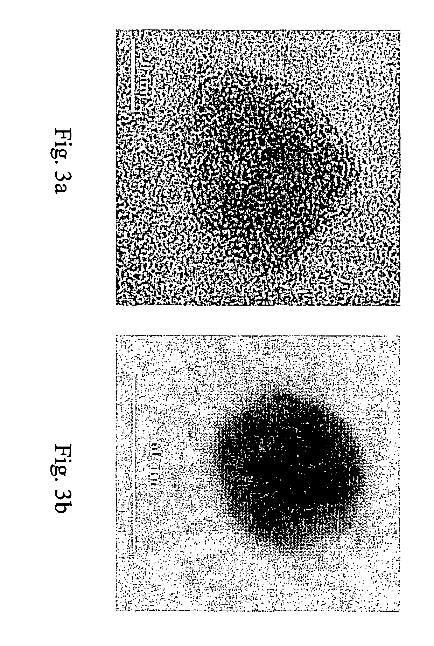

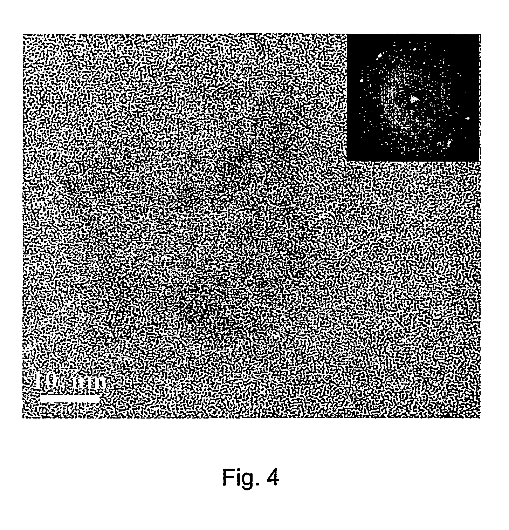

An electrostatic precipitator (ESP, InTox product) (7) was used to gather the particles on a carbon-coated copper grid (SPI Holley Carbon Grid). The morphology and crystallinity of the particles were then examined using a field emission transmission electron microscope (TEM, Philips CM200 FEG). Figures 3a and 3b show some TEM images of the particles. It is shown that the particles have curved surfaces with diameter of about 30-200 nm. The diffractogram of the particles, shown in Figure 4, indicates that the particle is crystalline.

### Example 2. Nano-sized naproxen particles

Naproxen is a non-steroidal anti-inflammatory analgesic agent used in the treatment of rheumatoid conditions. The naproxen liquid feed stock is atomized using a TSI 3076 aerosol generator, operating at solution flow rate of 0.1 to 2 ml/min. The resulted droplets, which are suspended into nitrogen gas, are then passed through a heated tube flow reactor. The oven temperature is set constant at a temperature of 150°C.

## Claims

1. A method for preparing free nano-sized particles of an active agent, comprising the steps of:
providing a liquid feed stock comprising an active agent or combination of two or more active agents;
atomising the liquid feed stock to create droplets;
suspending said droplets in a carrier gas;
passing said carried gas and droplets suspended therein through a heated tube flow reactor under predetermined residence time and temperature history; and
collecting the nano-sized particles produced,
wherein the mean mass aerodynamic diameter of the particles is less than 200nm.

2. A method according to claim 1 wherein the mean mass aerodynamic diameter of the particles is less than 100nm.

3. A method according to claim 1 wherein the particles are crystalline and have controlled particle size and shape.

4. A method according to any of claims 1 to 3, wherein the particles have a narrow particle size distribution with geometric standard deviation less than 2, preferably less than 1.5, most preferably less than 1.3

5. A method according to any of claims 1 to 4, wherein the particles are uncharged.

6. A method according to any of claims 1 to 5, wherein the particles are collected using a particle collection system selected from a group consisting of an electrostatic precipitator, a cyclone, a filter, a selling chamber, or an impactor.

7. A method according to any of claims 1 to 6 wherein the particle collection system is heated to a temperature above the dew point temperature of the solution to prevent condensation.

8. A method according to any of claims 1 to 7 wherein the liquid feed stock is in the form of a solution, suspension, dispersion, gel, emulsion, slurry or the like.

9. A method according to claim 8 wherein the solvent of the solution is water, hydrocarbon, halogenated hydrocarbon, alcohol or ketone.

10. A method according to any of claims 1 to 9 wherein the active agent is a therapeutic, cosmetic or diagnostic agent.

11. A method according to any of claims 1 to 10 wherein the active agent is a photochemical, catalyst, fertilizer, pigment, propellant, food, explosive or agricultural agent.

12. A method according to claim 10, wherein the therapeutic agent is an anti-inflammatory agent, a bronchodilating agent, an antibiotic agent, an antiviral agent, an immunosuppresive agent, an immunostimulatory agent, an antifungal agent, an anesthetic agent, an antioxidant, an antidiabetic agent, vitamin, hormone, antiepiletic agent, an anticancer agent, a muscle relaxant, an anti-HIV agent, stimulant, cough suppressant, pain control, smoking cessation, or anti alcohol abuse agent.

13. A method according to claim 12 wherein the anti-inflammatory agent is aspirin, naproxen, indomethacin, ibuprofen, fenoprogen, acetominophen, beclomethasone, diclofenac or a salt thereof.

14. A method according to claim 12 wherein the bronchodilating agent is salbutamol, salmeterol, formoterol, terbutaline, procaterol, or a salt thereof.

15. The method according to according to claim 10 wherein the active agent is a peptide, protein, steroid, nucleic acid, carbohydrate, lipid or radioactive compound.

16. A method according to any one of claims 1 to 15 in which the nano sized particles are formulated as dry powder, tablet, capsules, powder dispersed in liquid or colloidal suspension.

17. A method of making a pharmaceutical, cosmetic, diagnostic, photochemical, catalyst, fertilizer, pigment, propellant, food, explosive or agricultural composition comprising preparing nano-sized particles according to any one of claims 1 to 15.

## Patentansprüche

1. Verfahren zur Herstellung von Nanopartikeln eines aktiven Mittels, enthaltend die folgenden Schritte:
man stellt einen flüssigen Vorrat an Ausgangsmaterial, das ein aktives Mittel oder eine Kombination von zwei oder mehr aktiven Mittel enthält, zur Verfügung;
man zerkleinert das flüssige Ausgangsmaterial zur Erzeugung von Tröpfchen;
man suspendiert die Tröpfchen in einem Trägergas;
man leitet das Trägergas und die darin suspendierten Tröpfchen durch einen erhitzten Rohrfließreaktor bei vorgewählter Verweilzeit und Temperaturgeschichte; und
man sammelt die erzeugten Nanopartikel, worin der aerodynamische Durchmesser der mittleren Masse der Teilchen kleiner als 200 nm ist.

2. Verfahren nach Anspruch 1, wobei der aerodynamische Durchmesser der mittleren Masse der Teilchen kleiner als 100 nm ist.

3. Verfahren nach Anspruch 1, wobei die Teilchen kristalin sind und geregelte Teilchengröße und -form besitzen.

4. Verfahren nach einem der Ansprüche 1 bis 3, wobei die Teilchen eine enge Teilchengrößenverteilung mit geometrischer Standardabweichung von weniger als 2, vorzugsweise weniger als 1,5, insbesondere weniger als 1,3 besitzen.

5. Verfahren nach einem der Ansprüche 1 bis 4, wobei die Teilchen ungeladen sind.

6. Verfahren nach einem der Ansprüche 1 bis 5, wobei die Teilchen unter Verwendung eines Teilchensammelsystems aus der Gruppe bestehend aus einem elektrostatischen Abscheider, einem Zyklon, einem Filter, einer Absetzkammer oder einem Impaktor (Staubgehaltsprüfer) gesammelt werden.

7. Verfahren nach einem der Ansprüche 1 bis 6, wobei das Teilchensammelsystem auf eine Temperatur oberhalb der Taupunktstemperatur der Lösung zur Vermeidung von Kondensation erhitzt wird.

8. Verfahren nach einem der Ansprüche 1 bis 7, wobei der flüssige Vorrat an Ausgangsmaterial in Form einer Lösung, Suspension, Dispersion, eines Gels, einer Emulsion, Aufschlämmung oder ähnlichem vorliegt.

9. Verfahren nach Anspruch 8, wobei das Lösungsmittel der Lösung Wasser, Kohlenwasserstoff, halogenierter Kohlenwasserstoff, Alkohol oder Keton ist.

10. Verfahren nach einem der Ansprüche 1 bis 9, wobei das aktive Mittel ein therapeutisches, kosmetisches oder diagnostisches Mittel ist.

11. Verfahren nach einem der Ansprüche 1 bis 10, wobei das aktive Mittel eine Fotochemikalie, ein Katalysator, ein Düngemittel, Pigment, Treibstoff, Nahrungsmittel, Sprengstoff oder ein landwirtschaftliches Mittel ist.

12. Verfahren nach Anspruch 10, wobei das therapeutische Mittel ein entzündungshemmendes Mittel, ein Bronchienerweiterungsmittel, ein Antibiotikum, ein antivirales Mittel, ein Immunosupressivum, ein Immunostimulans, ein Fungizid, ein Anesthetikum, ein Antioxidans, ein Antidiabetikum, Vitamin, Hormon, Haarentfernungsmittel, ein Mittel gegen Krebs, ein Muskelrelaxans, ein Mittel gegen HIV, ein Stimulans, ein Mittel gegen Erkältung, ein Antischmerzmittel, Raucherentwöhnungsmittel oder ein Mittel gegen den Alkoholmissbrauch ist.

13. Verfahren nach Anspruch 12, wobei das entzündungshemmende Mittel Aspirin, Naproxen, Indomethacin, Ibuprofen, Fenoprogen, Acetominophen, Beclomethason, Diclofenac oder ein Salz hiervon ist.

14. Verfahren nach Anspruch 12, wobei das Bronchienerweiterungsmittel Salbutamol, Salmeterol, Formoterol, Terbutalin, Procaterol oder ein Salz hiervon ist.

15. Verfahren nach Anspruch 10, wobei das aktive Mittel ein Peptid, Protein, Steroid, eine Nukleinsäure, ein Kohlenwasserstoff, ein Lipid oder eine radioaktive Verbindung ist.

16. Verfahren nach einem der Ansprüche 1 bis 15, wobei die Nanopartikel als trockenes Pulver, Tablette, Kapsel, in Flüssigkeit dispergiertes Pulver oder als kolloidale Suspension formuliert werden.

17. Verfahren zur Herstellung eines pharmazeutischen, kosmetischen, diagnostischen, fotochemischen, Katalysator-, Dünger-, Pigment-, Treibstoff-, Nahrungs-, Sprengstoff- oder eines landwirtschaftlichen Mittels, das die Herstellung von Nanopartikeln nach einem der Ansprüche 1 bis 15 umfasst.

## Revendications

1. Procédé de préparation de nanoparticules libres d'un principe actif, comprenant les étapes de :
fournir une charge liquide comprenant un principe actif ou une combinaison de deux principes actifs ou plus ;
atomiser la charge liquide pour créer des gouttelettes ;
suspendre lesdites gouttelettes dans un gaz vecteur ;
faire passer ledit gaz vecteur et les gouttelettes suspendues dans celui-ci à travers un réacteur continu à tube chauffé pendant un temps de séjour et un historique de températures prédéterminé ; et
recueillir les nanoparticules produites,
dans lequel le diamètre aérodynamique massique médian des particules est inférieur à 200 nm.

2. Procédé selon la revendication 1, dans lequel le diamètre aérodynamique massique médian des particules est inférieur à 100 nm.

3. Procédé selon la revendication 1, dans lequel les particules sont cristallines et ont une taille et une forme de particule contrôlées.

4. Procédé selon l'une quelconque des revendications 1 à 3, dans lequel les particules ont une granulométrie des particules étroite avec une déviation standard géométrique inférieure à 2, de préférence inférieure à 1,5, plus préférablement inférieure à 1,3.

5. Procédé selon l'une quelconque des revendications 1 à 4, dans lequel les particules ne sont pas chargées.

6. Procédé selon l'une quelconque des revendications 1 à 5, dans lequel les particules sont recueillies en utilisant un système collecteur de particules choisi dans le groupe constitué par un précipitateur électrostatique, un cyclone, un filtre, une chambre ou un impacteur.

7. Procédé selon l'une quelconque des revendications 1 à 6, dans lequel le système collecteur de particules est chauffé à une température au-dessus du point de rosée de la solution pour empêcher la condensation.

8. Procédé selon l'une quelconque des revendications 1 à 7, dans lequel la charge liquide est sous la forme d'une solution, d'une suspension, d'une dispersion, d'un gel, d'une émulsion, d'une pâte ou analogue.

9. Procédé selon la revendication 8, dans lequel le solvant de la solution est l'eau, un hydrocarbure, un hydrocarbure halogéné, un alcool ou une cétone.

10. Procédé selon l'une quelconque des revendications 1 à 9, dans lequel le principe actif est un agent thérapeutique, cosmétique ou de diagnostic.

11. Procédé selon l'une quelconque des revendications 1 à 10, dans lequel le principe actif est un agent photochimique, un catalyseur, un engrais, un pigment, un propulseur, un aliment, un agent explosif ou un agent agricole.

12. Procédé selon la revendication 10, dans lequel l'agent thérapeutique est un agent anti-inflammatoire, un agent bronchodilatateur, un agent antibiotique, un agent antiviral, un agent immunosuppressif, un agent immunostimulateur, un agent antifongique, un agent anesthésique, un antioxydant, un agent antidiabétique, une vitamine, une hormone, un agent antiépileptique, un agent anticancéreux, un myorelaxant, un agent anti-VIH, un stimulant, un antitussif, un agent de contrôle de la douleur, un agent de désaccoutumance au tabac, ou un agent antialcoolisme.

13. Procédé selon la revendication 12, dans lequel l'agent anti-inflammatoire est l'aspirine, le naproxène, l'indométhacine, l'ibuprofène, le fénoprogène, l'acétominophène, la béclométhasone, le diclofénac ou un sel de ceux-ci.

14. Procédé selon la revendication 12, dans lequel l'agent bronchodilatateur est le salbutamol, le salmétérol, le formotérol, la terbutaline, le procatérol, ou un sel de ceux-ci.

15. Procédé selon la revendication 10, dans lequel le principe actif est un peptide, une protéine, un stéroïde, un acide nucléique, un glucide, un lipide ou un composé radioactif.

16. Procédé selon l'une quelconque des revendications 1 à 15, dans lequel les nanoparticules sont formulées sous la forme de poudre sèche, comprimés, gélules, poudre dispersée dans un liquide ou une suspension colloïdale.

17. Procédé de préparation d'une composition pharmaceutique, cosmétique, de diagnostic, photochimique, catalytique, d'engrais, de pigment, de propulseur, alimentaire, explosive ou agricole comprenant la préparation de nanoparticules selon l'une quelconque des revendications 1 à 15.
